# EUROPEAN PATENT APPLICATION

(11) **EP 4 782 823 A1**
(43) Date of publication of application: **29.07.2026**
(21) Application number: 26151962.3
(22) Date of filing: 15.01.2026
(51) Int. Cl.: G01N 23/04, G01N 23/083, G01N 23/16, G01N 23/18, G01N 33/00

(54) **APPARATUS AND METHOD FOR CHECKING, BY MEANS OF ARTIFICIAL INTELLIGENCE, THE INTEGRITY OF RADIOPROTECTIVE PERSONAL PROTECTIVE EQUIPMENT AGAINST IONIZING RADIATIONS**

(30) Priority: 23.01.2025 IT 202500001209
(71) Applicant: Simad S.r.l., 40066 Pieve di Cento (BO) (IT)
(72) Inventor: FALLAVENA, Franco, 40066 Pieve di Cento (BO) (IT)
(74) Representative: Gregorj S.r.l.

(57) **Abstract**

The present invention refers to an apparatus (1, 1') for checking, by means of artificial intelligence, the integrity of radioprotective personal protective equipment (21, 21', 21"). The apparatus (1, 1') comprises: a checking chamber (2), personal protective equipment supporting means (3, 3'), X-ray emission means (4), X-ray detection means (5), a control unit (6) implementing artificial intelligence. The control unit (6) is configured to perform the following operations:
∘ obtaining, by artificial intelligence, an index of suitability (IN) for the radioprotective personal protective equipment,
∘ based on the obtained index of suitability (IN), assessing, preferably by means of artificial intelligence, whether the radioprotective personal protective equipment is defective or potentially defective.

## Description

### Technical field of the invention

The present invention refers to an apparatus and method of checking, by means of artificial intelligence, the integrity of personal protection equipment against ionizing radiations.

The wording "personal protective equipment" means equipment destined to be worn by an individual to protect him/her from one or more risks susceptible to impair the safety or health, and also any complement or accessory destined to this purpose. Particularly, the present invention refers to personal protective equipment against ionizing radiations.

Ionizing radiations are radiations which have an energy sufficient to directly or indirectly ionize the atoms or the molecules which they interact with, so as to determine alterations of the electron structure of them. Such radiations are particularly dangerous if an individual is subjected to a repeated prolonged exposure. In fact, they can affect the biological tissues, determining biological damages which can be also serious, particularly tumors. Examples of ionizing radiations are α-rays, β-rays, γ-rays, and X-rays.

Referring particularly to X-rays, they are exploited, as it is known, in medical environments (such as hospitals, medical offices, clinics, dentist's offices) for diagnostic purposes, for example for taking radiographies. Moreover, artificial radioactive sources for therapeutical and diagnostic purposes are used.

### Prior art and technical problem

Since the ionizing radiations are potentially dangerous, it is essential that, when radiographies are carried out, the X-rays hit only those portions of the body of an individual on which a radiologic probe must be performed. Therefore, the zones of the body of the individual/patient which are not involved in the radiography must be protected.

A particularly perceived requirement is one that consists of protecting, for example, the operators, the radiology operators, technicians, doctors from the X-ray exposure. In fact, they, due to their professions, are repeatedly exposed to radiations, therefore it is essential to provide systems adapted to protect them.

In order to protect patients and operators, using suitable personal protective equipment is known. Such personal protective equipment comprises a core made of a radiation shielding material (shielding lamina). For example, they are made of lead, which is particularly adapted to the X-ray protection, or are made of lead rubber or materials alternative to lead for manufacturing the ionizing radiation protection equipment.

Personal protective equipment can be damaged by improper use or simply by wear caused by a prolonged use. For example, cracks or slits can be formed in their protective core. In such cases, the equipment is no longer capable of suitably protecting an individual.

Therefore, methodically and accurately checking the integrity status of the personal protective equipment is a requirement, this is performed by subjecting the equipment to radiations of the type which they must protect against, in order to find possible slits or cracks.

Some types of personal protective equipment are characterized by complicated shapes. For example, in some types, plural layers of shielding materials are overlapped on each other, in order to increase the protection degree. Therefore, checking the integrity which must involve all the single layers, is rather burdensome. Moreover, in order to correctly assess the integrity, the different portions of the personal protective equipment are required to be correctly positioned with respect to the beam of X-rays, particularly with respect to the source and intensifier of the X-ray machine used for checking, this operation is not always easy if the personal protective equipment has a complicated shape.

Further, checking the integrity must be carefully performed, in order to not cause damages to the structure of the personal protective equipment. In fact, improper mechanical actions could damage the personal protective equipment.

Therefore, it is important to check the integrity of the personal protective equipment. To this purpose, the apparatus and method of the Italian patent No. 1406715, in the name of the same Applicant of the present patent application, are known.

The Applicant has noted that the apparatus and method of the above cited patent can be further improved and therefore proposes the apparatus and method described hereinafter.

### Objects of the invention

Therefore, the object of the present invention consists of improving and making more efficient the integrity assessment of personal protective equipment manufactured according to the prior art.

Therefore, an object of the present invention consists of providing an apparatus and method enabling to optimize and expediting the integrity check of personal protective equipment.

A further object of the invention consists of reducing the errors when the integrity of personal protective equipment is assessed.

Further, the invention intends to provide an apparatus and a method enabling to monitor the integrity of personal protective equipment also by an unskilled operator.

These and other objects are met by apparatuses and methods according to the following description, the attached claims and the following aspects.

### Summary of the invention

Aspects of the invention, which are an integral part of the technical content of the present patent text, are herein described. Said aspects can be used to limit the claims and/or define further claims during the life of the present patent protection.

The invention provides the use of artificial intelligence for monitoring the integrity of personal protective equipment and thus detecting possible defects.

Artificial intelligence operates on a dataset, comprising a group of images of personal protective equipment; the dataset provides images obtained by integrity tests on personal protective equipment performed and validated by qualified experts. For example, the dataset can provide images of integrity tests performed by using the apparatus and method of the Italian patent No. 1406715.

The invention provides also a method of monitoring, by means of artificial intelligence, the integrity of personal protective equipment and thus detecting possible defects, by using the apparatus.

### Conventions and definitions

It is noted that in the following detailed description corresponding parts/components/elements are indicated by the same numeral references. The figures could illustrate the object of the invention by not-to-scale representations; therefore, parts/components/elements illustrated in the attached figures and regarding the object of the invention, could only refer to schematic representations. Using "/" in the present text means "and/or", if it is not clearly indicated otherwise or unless otherwise specified.

In the context of the present disclosure, the use of terms such as "on", "top", "upper", "below", "bottom", "lower", "alongside", "lateral", "laterally", "internal", "internally", "external", "externally", "horizontal", "horizontally", "vertical", "vertically", "frontal", "frontally", "posterior", "posteriorly", "right", "left", similar terms and corresponding variants refer, except for specific different indications, to at least one spatial orientation that the object of the invention can take under conditions of use; the terms refer to what the drawings show, illustrating the orientation associated to a normal use of the invention. Such terms are used only for helping the reader and are not limiting.

Except for specific different indications, the terms "condition" or "configuration" can be interchangeably used in the context of the present disclosure. The expression "said at least one" is interchangeable with "each". The expression "each" and similar do not necessarily imply that it is referred to a plurality of elements; for example, "each element" can refer to just one element or to a plurality of elements, based on the context where it is used and/or based on the embodiment which it refers to.

In the context of the present disclosure one or more of the following definitions/conventions, when required and unless otherwise indicated and/or unless otherwise excluded by the context, can be applied:
- hereinafter, "personal protective equipment" can be simply indicated by PPE;
- "radioprotective personal protective equipment" means personal protective equipment against X type ionizing radiations;
- the radioprotective personal protective equipment is configured to protect the eyes (second embodiment) and/or at least another part of the body of a user of the PPE (first embodiment);
- examples of PPE whose integrity can be checked by the apparatus and method according to the invention are: aprons, skirts, vests, full aprons, collars, gonad protection elements, ovary protection elements, gloves, arm covers, aprons and skirts for kids, thyroid protection elements, shrugs, hand covers, aprons for intraoral exams (the integrity of all the beforehand listed PPE can be checked by the apparatus according to the first embodiment), glasses or masks or other radioprotective PPE wearable for protecting the eyes of a user (the integrity of glasses or masks or other PPE adapted to protect the eyes can be checked by the apparatus according to the second embodiment). Each PPE can be dedicated based on the part of the body which must be protected;
- the expressions "lighter color" and "darker color" must be understood with reference to each other; therefore, the lighter color cannot be a light color in absolute terms, as long as it is lighter than the darker color and, viceversa, the darker color cannot be a dark color in absolute terms, as long as it is darker than the darker color. Such colors are compatible with the colorations typical of fluoroscopic images. Preferably, the darker color is dark grey/black, and the lighter color is white/light gray.

Said conventions and definitions can be used, when required, to interpret the claims. As required, one or more of said conventions and definitions can be included in one or more of the following claims and/or in one or more of the preceding aspects, particularly when these claims and/or aspects use one or more expressions object of one or more conventions or definitions.

### Control unit

The apparatus for checking, by means of artificial intelligence, the integrity of radioprotective personal protective equipment herein disclosed can comprise at least one control unit capable of controlling/guiding/managing the operations/steps of the apparatus and therefore the conditions of use implemented by the apparatus itself. In the same way, the method of checking, by means of artificial intelligence, the integrity of radioprotective personal protective equipment herein disclosed can use at least one control unit capable of controlling/guiding/managing the steps of the method and, in addition or as an alternative to, is capable of controlling/managing the conditions of use implemented by the apparatus.

The control unit can be computerized and/or controllable by a computer or another control device. The control unit can be a single control unit or can include a plurality of distinct control units, according to design choices and according to operative requirements destined to implement the invention.

The term "control unit" indicates an electronic component which can comprise at least one among: a digital processor (CPU), an analogic circuit, or a combination of one or more digital processors with one or more analogic circuits. The control unit can be "configured" and/or "programmed" to execute some steps: this can be done by any means and/or modes enabling to configure and/or program the control unit. For example, in the case of a control unit comprising one or more CPUs and one or more memories, one or more programs or software can be stored into suitable memory banks connected to the CPU/CPUs; the program or programs contain instructions which, when implemented by the CPU/CPUs, program or configure the control unit to execute the operations herein described with reference to the control unit. Alternatively, if the control unit provides circuitry and/or analogic hardware, the circuit of the control unit can be designed to include the circuitry and/or hardware configured, in use, to process electrical signals in order to execute the steps regarding the control unit.

The control unit implements (in other words uses) artificial intelligence, as hereinafter described.

### Brief description of the drawings

In order to better understand the invention and appreciate the advantages, some embodiments thereof will be described in the following in an exemplifying and non-limiting way with reference to the attached figures.

A brief description of the attached figures follows:

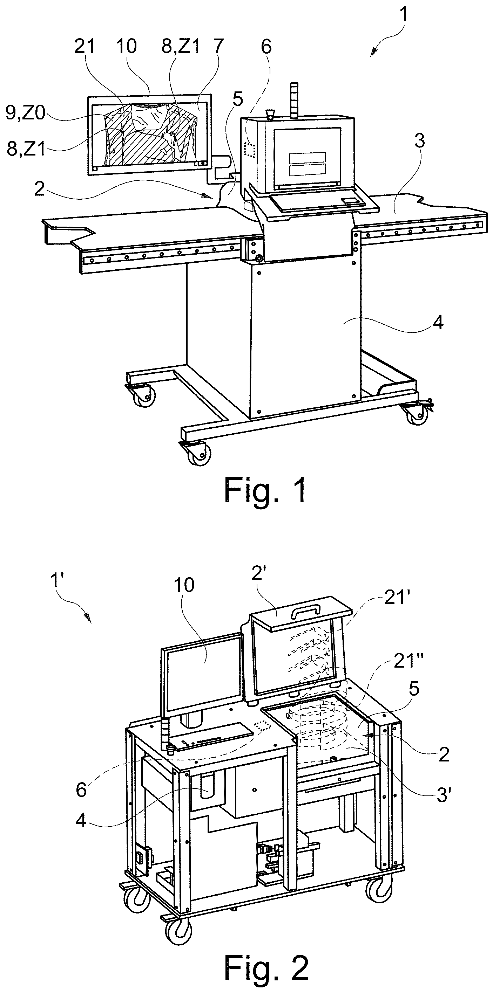

Figure 1 illustrates a first embodiment of the apparatus according to the invention. According to the first embodiment, the apparatus is configured to check the integrity of radioprotective personal protective equipment destined to protect one or more parts, eyes excluded, of the human body of a user;
Figure 2 illustrates a second embodiment of the apparatus according to the invention. According to the second embodiment, the apparatus is configured to check the integrity of radioprotective personal protective equipment destined to protect the eyes of a user. Examples of such PPE are a pair of glasses or a mask;

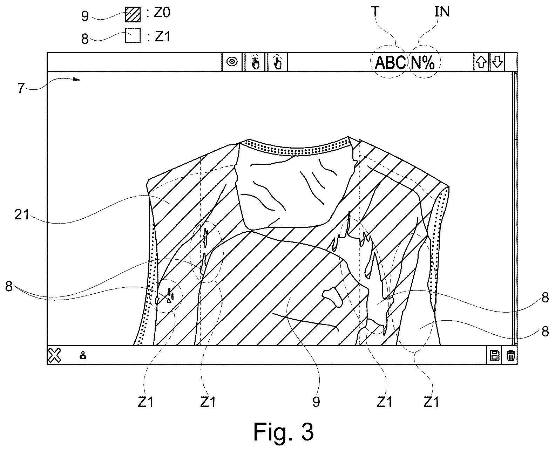

Figure 3 illustrates an image of the PPE made to assess its integrity. More particularly, the image of Figure 3 is of the type of that displayed on the screen of the apparatus of Figure 1.

### Detailed description of embodiments of the invention

### Apparatus

An apparatus according to the invention is generally indicated in the figures by the numeral reference 1. The apparatus 1, 1' is configured to check, by means of artificial intelligence, the integrity of radioprotective personal protective equipment, such as an apron 21 (see Figure 1), glasses 21' and a mask 21" (see Figure 2).

The apparatus 1, 1' comprises:
- a checking chamber 2, within which the PPE is positioned, in order to control its integrity,
- means 3, 3' for supporting personal protective equipment configured to support at least one radioprotective PPE 21, 21', 21" within the checking chamber 2,
- X-ray emitting means 4,
- X-ray detection means 5 (X-ray detector),
- a control unit 6, preferably configured to implement artificial intelligence.

The checking chamber 2 can provide an open/closure element, such as a door 2', for opening/closing an access to the checking chamber 2 and therefore to enable/prevent the access to the checking chamber 2.

Advantageously, the PPE supporting means 3, 3' are located within the checking chamber 2 and provide a supporting plane 3, 3', which is configured to support the PPE 21, 21', 21" and is preferably movable. The relative movement between the PPE and the supporting plane 3, 3' enables to obtain a correct relative positioning between the PPE and the supporting plane in order to correctly locate the PPE within the checking chamber 2. It is noted that, in Figure 2, the glasses 21' and the mask 21" are represented by broken lines to indicate their proximity to the supporting means 3, 3'. The supporting means 3, 3' can provide, besides the supporting plane 3', 3', a suitable support for abutting on the glasses 21' and the masks 21", which can be made in a single piece (integrated, for example by 3D printing) piece with the supporting plane 3, 3'.

According to the first embodiment of the apparatus 1, illustrated in Figure 1, the supporting plane 3 is sliding; therefore, the PPE abuts on the supporting plane which can slide. The apparatus 1 according to the first embodiment, is devised to analyze the integrity of PPE 21 (except for glasses and masks). The supporting plane 3 translates based on the size of the PPE 21; for example, the PPE 21 can be bigger, also much bigger, than the X-ray detector 5; in this case, according to the first embodiment of the apparatus 1 and method, it acquires the entire image of the PPE 21 by translating this latter and the supporting plane 3 which supports it, then by composing/assembling the final image based on the single acquired images 7. It is noted that the apron 21, the integrity thereof is to be assessed by means of the apparatus 1 of Figure 1 and part of it is illustrated in the image 7 visible in Figure 1, has dimensions much greater than the ones of the X-ray detector 5 and therefore it acquires a plurality of images 7 for assessing it as a whole.

According to the second embodiment of the apparatus 1', illustrated in Figure 2, the supporting plane 3' is rotatable; therefore, the PPE abuts, for example on one or more suitable supports configured to position the glasses 21' or the mask 21" in a predetermined position, on the supporting plane 3 which can rotate. The apparatus 1' according to the second embodiment, is devised to analyze the integrity of PPE 21', 21" for protecting the eyes (glasses, masks and analogous PPE). In the second embodiment, PPE 21', 21" is as big as or less big than the X-ray detector (see Fig-ure 2); the rotation of the supporting plane 3' is configured, and is used, to observe the PPE 21', 21" from different angulations. This enables to optimally assess the integrity of the PPE 21', 21".

The control unit 6 is configured to perform, under conditions of use of the apparatus 1, 1' wherein a radioprotective PPE 21, 21', 21" is supported on the supporting means 3, 3', the following operations:
o obtaining, by means of artificial intelligence, an index of suitability IN for the radioprotective PPE,
o based on the obtained index of suitability IN, assessing whether the radioprotective PPE 21, 21', 21" is defective or potentially defective.

Assessing whether the radioprotective PPE is defective or potentially defective is preferably made by artificial intelligence; alternatively, it can be done by an operator.

Moreover, the control unit 6 is configured to perform the following operations:
- analyzing from a chromatic point of view at least one image 7 or a series of images of the radioprotective PPE, the one or more images being acquired during an X-ray emission towards the radioprotective PPE, each image being preferably a fluoroscopic image,
- detecting on the image/s 7 at least a difference in color of the radioprotective PPE 21, 21', 21".

Preferably, the color difference is given by the presence of a different color 8, particularly a lighter color 8, in or within or close to a zone (or area) Z0 of the image of the radioprotective PPE having another color or predominantly another color 9, particularly a darker color 9. As illustrated in Figure 3, the lighter color 8 is defined in the zones Z1, while the darker color 9, outlined by a broken line, is defined in the zones Z0.

More particularly, the control unit 6 is configured to detect on the image/s 7 at least a color difference given by the presence of a lighter color (first color) 8 in or within or close to a zone Z1 of the radioprotective PPE having another color or predominantly another color (second color) 9 and/or in an adjacent zone (Z0) of the radioprotective PPE. The other color 9 is darker than the lighter color 8. As indicated and outlined in Figure 3 in the zones Z1, the differences/variations of color are light grey/white 8 (with respect to the adjacent zone Z0 of dark grey/black/another dark color 9), based on the zone or area of the PPE being looked at.

The color difference 8, 9 between adjacent zones Z1, Z0 of the radioprotective PPE is an indication to be considered in assessing the presence of substantial breakages/damages in the PPE.

The analysis assessing the integrity of the PPE can be performed on a plurality of images 7. The plurality or series of images 7 can be relative to different portions of a same PPE 21, 21', 21" and/or to different angulations of the same PPE 21, 21', 21". Moreover, the images 7 can be composed/assembled, for example by and/or under supervision of the control unit 6, in order to enable to assess the integrity of the PPE 21 with reference to its entirety. Consequently, the control unit 6 is preferably configured to perform the following operations by means of artificial intelligence:
o analyzing from a chromatic point of view a plurality of images 7 of one or more radioprotective PPE,
o classifying the plurality of images 7 after analyzing them from a chromatic point of view.

The apparatus 1, 1' further comprises an image acquisition and/or recording member, configured to acquire, under conditions of use, one or more images 7 of the radioprotective PPE. Preferably, the X-ray detection means 5 comprise the image acquisition and/or recording member. Starting from the image/s acquired in this way, the following processing is performed. In a preferred embodiment, the image acquisition and/or recording member is a detector (known as "Flat Panel"), wherein the sensor elements are formed by a thin layer of amorphous silicon, and which provides a scintillator and a photosensor. Once the X-ray beam hits its surface, a signal builds up on the capacitor to a desired value. The scintillator, usually based on cesium iodide, converts the X-ray into an optical signal. The photosensor, formed by photodiodes and switching systems, converts into an electrical signal what was generated by the scintillator, consequently enabling it to obtain a radiographic image (fluoroscopic image).

Preferably, the apparatus 1, 1' comprises also a display support 10 on which an operator can display, particularly monitor, whether the radioprotective PPE is defective or potentially defective based on information provided by means of artificial intelligence. In the illustrated figures, the display support is in the form of a screen 10.

### Training, artificial intelligence, and neural network

The control unit 6 is prepared to perform the beforehand cited operations based on the integrity assessment of the PPE; this is made possible by artificial intelligence. In fact, the control unit 6 implements artificial intelligence operating on a dataset developed by beforehand performed and validated tests; the control unit 6 is trained by the dataset. The dataset comprises a group of images of personal protective equipment; the dataset provides images obtained by integrity tests on personal protective equipment performed and validated by qualified experts. During the development step of the apparatus performed by the Applicant, the dataset is given by the group of images related to beforehand performed controls which were validated by skilled personnel of the Applicant based on the degree of suitability of the PPE; artificial intelligence was trained by the dataset, in order to generate a neural network model.

More particularly, it is noted that the model was generated by the algorithm *FastTreeBinaryTrainer* during the training based on the custom dataset, the architecture of the neural network is of a convolutional type (*Convolutional Neural Network,* known as CNN) selected because this was identified as the type of neural network more efficient for processing images and grid data patterns. More particularly, the neural network has been realized/optimized by using the packet ML.net (a model of Machine Learning by Microsoft^{®}) by a classifying algorithm FastTreeBinaryTrainer. The model is executed by the library ML.NET with a code written in the language C# and locally executed on a CPU (control unit). Clearly, in other embodiments, variants of models, algorithms and/or neural networks can be used, of course with the required training.

Artificial intelligence operates by classifying the images of the new controls in order to obtain an index of suitability by percentage IN (see Figure 3). The classification is performed by analyzing the images following some steps, particularly six typical steps of the CNN; the purpose of the generated model is to obtain a suitability classification index which varies between 0% and 100%.

### Analyzing the images of the PPE

When the index of suitability IN, as a function of its value, indicates that an unsuitable PPE is displayed, a visual research of the damages is performed based on the color difference 8, 9 between zones Z1, Z0 that are close, for delimiting areas characterized by substantial differences. A determined limit value between suitability and unsuitability of PPE can be provided for assessing/cataloging the PPE itself, for example an intermediate value (e.g. 50%) between the extremes of a scale of values (for example 0% and 100%) which the index of suitability IN can have. Since the index of suitability IN can be expressed as a percentage, the PPE can be identified as unsuitable (defective/broken) when the index is less than 50%, while when this latter is more than 50%, the PPE is identified as suitable.

The research is automatically performed (in other words by artificial intelligence) on the image, by assessing the presence of a light color 8, the average difference thereof is greater than a value x with respect to the zone Z0 of a darker color 9 which contains it and viceversa. Substantially, a calculation of the average of the color zone by adding the same value of the color and dividing it by the number of pixels composing the zone is done; the wording average difference means the difference between the average of two zones as hereinbefore indicated. The color difference 8, 9 is generally unusual and is the index of unsuitability if it has a dimension greater than a predetermined certain dimension (measurement), preferably expressed by square millimeters, for example obtained by multiplying the physical dimension of the pixel for the number of pixels within the involved zone Z0, Z1.

### Indicating and graphically displaying whether the PPE is suitable or not

Therefore, artificial intelligence enables to classify the status (integrity or not) of the PPE and to provide the percentage (index IN) of integrity of the PPE, in order to give to a human operator using the apparatus 1, 1', also in case of an unskilled operator, an instrument useful to increase the efficiency and quality of his/her work and to improve the safety for a user of the radioprotective PPE. However, an indication T, for example a text (shown in Figure 3 by ABC), indicating the status of integrity of the PPE, can be provided on the image 7. Such indication or text T can have the following form: "indicatively suitable/unsuitable by N%", wherein N% is a determined index of suitability.

Therefore, the apparatus 1, 1' enables an automatic and fast research of the defects detected by artificial intelligence; if a PPE is not found integral, the operator receives a control feedback. Such operation is performed by measuring the defective area/zone Z1 with reference to the one of the whole PPE, making accurate the identification and reducing in this way a possible error which the operator himself/herself could be subjected to. The visual indication of the index of suitability is presented so that is always visible, for example at the right top of the image 7, preferably by a red color (unsuitable) or green (suitable), when displaying the image 7. The red color and green color can be derived from the indication or text T displayable on the image 7, as a function of the value of the index of suitability IN. Consequently, in addition to the index of suitability IN, the control unit 6 provides also an indication or text T and/or a chromatic indicator (for example, red/green) for enabling a skilled or unskilled operator to immediately and simply identify whether the PPE is suitable or not. On the screen 10, the image 7 can scroll by an input from the operator, for example for obtaining further detailed assessments, etcetera.

The hereinbefore described operations, particularly performed by the control unit 6, can be steps of the method described in the following.

### Use

Further, the invention refers to a use of the apparatus 1, 1' for checking the integrity of radioprotective PPE 21, 21', 21". The use of the apparatus 1, 1' is further explained in the description of the method and of the specific steps.

### Method

Moreover, the present invention refers to a method for checking, by artificial intelligence, the integrity of radioprotective PPE 21, 21', 21". Preferably, the method is performed by means of the beforehand described apparatus 1, 1'; in such case, the method provides the step of predisposing an apparatus 1, 1' of the beforehand described type, wherein radioprotective personal protective equipment 21, 21', 21" is supported by the support means 3, 3'.

The method provides the steps of:
- emitting X-rays so that they interact with the radioprotective personal protective equipment 21, 21', 21" located within a checking chamber (2),
- detecting the status of integrity of the radioprotective personal protective equipment 21, 21', 21", preferably after the interaction of the X-rays with the radioprotective personal protective equipment 21, 21', 21".

The step of detecting the status of integrity of the radioprotective PPE comprises the following steps:
o obtaining, by artificial intelligence, an index of suitability IN for the radioprotective PPE,
o based on the obtained index of suitability IN, preferably assessing by artificial intelligence, whether the radioprotective PPE is defective or potentially defective.

Preferably, the step of emitting X-rays provides to activate the X-ray emission by the X-rays emission means 4.

Preferably, the step of detecting the status of integrity provides to detect the status of integrity of the radioprotective personal protective equipment 21, 21', 21" by detecting the X-rays by the X-rays detection means 5 after the interaction of the X-rays with the radioprotective personal protective equipment 21, 21', 21".

The method can provide the step of positioning PPE on the supporting means 3, 3'. The supporting means 3, 3' can be moved and/or positioned, particularly for ensuring a correct positioning of the PPE. In order to position the PPE and/or the support means themselves with respect to it, the method can provide the movement of the support means 3, 3', by translation in the first embodiment and by rotation in the second embodiment. The position of the PPE 21, 21', 21" can provide the step of abutting the PPE 21, 21', 21" on the support plane 3, 3', preferably by a suitable support.

As hereinbefore said, according to the second embodiment, the PPE 21', 21" is configured to protect the eyes of a user, while according to the first embodiment the PPE 21 is configured to protect another part of the body (different from the eyes) of a user.

Moreover, the method provides the steps of:
- analyzing from a chromatic point of view at least one image 7 or a series of images of the radioprotective PPE, the one or more images being acquired during the X-ray emission towards the radioprotective PPE, each image being preferably a fluoroscopic image,
- detecting on the image/s at least one color difference of the radioprotective PPE, preferably the color difference being given by the presence of a different color 8, for example a lighter color in or within or close to a zone Z0 of the image of the radioprotective PPE having another color or predominantly another color 9, for example a darker color.

Preferably, the method provides to:
o analyze from a chromatic point of view, a plurality of images 7 of one or more radioprotective PPE,
o classify the plurality of images 7 after analyzing them from a chromatic point of view.

As hereinbefore described with reference to the control unit 6, the method provides to detect on the image/s at least a color difference, the color difference being given by the presence of a lighter color 8 in a zone Z0 having another color or predominantly another color 9 and/or in an adjacent zone of the radioprotective PPE, said other color 9 being darker than the lighter color 8. The zone having the lighter color 8 is indicated in Figure 3 by Z1 and is defined within a zone Z0 having a darker color 9.

Before analyzing the image/s, the method provides to acquire and/or record at least one image or a series of images, particularly of the fluoroscopic type of the radioprotective PPE during the X-ray emission towards the radioprotective PPE, the PPE being positioned within the checking chamber 2. The X-ray emission is necessary for acquiring the fluoroscopic image of the PPE for determining the integrity of the PPE, particularly of the shielding lamina of the PPE. From the image/s acquired during the detection step by the X-ray emission, artificial intelligence assesses the image for defining the index of suitability of the PPE. Such index IN varies from 0% to 100%, preferably in a unitary way, in other words by variations of 1% from 0% to 100% (without values with a decimal point, or decimal numbers, between integers consecutive to each other). The color difference 8, 9 between adjacent zones Z1, Z0 of the PPE is an indication which is considered for assessing the presence of substantial damages to the PPE.

According to the first embodiment, the method is for checking, by artificial intelligence, the integrity of radioprotective PPE wearable for protecting one or more parts of the body of a user of the PPE, the parts of the body being different from eyes.

According to the second embodiment, the method is for checking, by artificial intelligence, the integrity of glasses or masks or other radioprotective PPE 21', 21" wearable for protecting the eyes of a user.

The persons skilled in the art will recognize that, unless otherwise indicated in the present disclosure, the particular sequence of described steps is simply illustrative and can be changed still falling in the scope of what is herein disclosed and protected by the attached claims. Therefore, unless otherwise indicated or except for references to specific elements/steps by the use of the same terms, the method steps can be performed with any convenient or desirable order.

### Further advantages and conclusive notes

The invention enables, thanks to artificial intelligence, to provide an apparatus 1, 1' and a method capable of expediting and automatizing the integrity check of radioprotective PPE, by simultaneously reducing the integrity assessment errors. Moreover, artificial intelligence applied to the apparatus 1, 1' and the method increases the efficiency and quality of the work of the (skilled or unskilled) human operator operating on the apparatus 1, 1' because it provides, by the index of suitability IN, an indication capable of expediting the work of the operator. Substantially, the operator can more quickly assess whether PPE 21, 21', 21" is suitable or not based on the value of the index of suitability IN, particularly if it is close to or very close to the extremes of the scale of values (in case of an index of suitability IN expressed by a percentage, to 0% or 100%).

Advantageously, the invention enables, thanks to artificial intelligence, also an unskilled human operator, to determine whether the PPE is integral or not, by making feasible to rapidly determine possible breaks/damages of the shielding lamina and reducing labor costs (because skilled technicians or personnel are not required and because the assessment of the integrity is expedited), with a consequent increase of the work performance and of the efficacy. The labor costs are reduced by the apparatus or by the method according to the invention because skilled technicians or personnel are not necessarily required and because the assessment of integrity is expedited. The Applicant has statistically observed, by tests performed by unskilled personnel, a reduction of about 40% of the human error by artificial intelligence (with reference to the acknowledgement of the defects of the radioprotective PPE), therefore enabling to increase the quality and amount of the labor together with a greater safety to the health of the users of the radioprotective PPE.

The protection given by the claims extends to each element, component and/or step of the invention equivalent to what is claimed.

It is understood that each element, component and/or step of the product/method according to the invention can be substituted with an equivalent element, component and/or step (in the following, "equivalent"); such equivalent/s can be already existent at the filing or priority date of the present patent text or can be of subsequent conception/development.

## Claims

1. Apparatus (1, 1') for checking, by means of artificial intelligence, the integrity of radioprotective personal protective equipment (21, 21', 21"), the apparatus comprising:
- a checking chamber (2)
- means (3, 3') for supporting personal protective equipment, the supporting means (3, 3') being configured to support at least one radioprotective personal protective equipment (21, 21', 21") within the checking chamber (2),
- X-ray emitting means (4)
- X-ray detection means (5), preferably the X-ray detection means (5) being configured to acquire one or more images of the radioprotective personal protective equipment (21, 21', 21"),
- a control unit (6) implementing artificial intelligence,
wherein the control unit (6) is configured to perform, under conditions of use in which a radioprotective personal protective equipment (21, 21', 21") is supported by the supporting means (3, 3'), the following operations:
∘ obtain, by means of artificial intelligence, an index of suitability (IN) for the radioprotective personal protective equipment (21, 21', 21"),
∘ on the basis of the suitability index (IN) obtained, assessing, preferably by means of artificial intelligence, whether the radioprotective personal protective equipment (21, 21', 21") is defective or potentially defective.

2. Apparatus according to claim 1, wherein the control unit (6) is configured to perform the following operations:
- analysing from a chromatic point of view at least one image (7) or a series of images of the radioprotective personal protective equipment (21, 21', 21"), the one or more images being acquired during X-ray emission towards the radioprotective personal protective equipment (21, 21', 21"), each image preferably being a fluoroscopic image (7),
- detecting on said at least one image (7) at least one color difference (8, 9) of the radioprotective personal protective equipment (21, 21', 21"), preferably the color difference (8, 9) being due to the presence of a different color (8), e.g. a lighter color, in or within or near an area (Z1, Z0) of the image (7) of the radioprotective personal protective equipment (21, 21', 21") having another color or predominantly another color (9), e.g. a darker color.

3. Apparatus according to claim 2, wherein the control unit (6) is configured to perform the following operations using artificial intelligence:
o analysing from a chromatic point of view a plurality of images (7) of one or more radioprotective personal protective equipment (21, 21', 21"),
o classifying said plurality of images (7) after analysing them from a chromatic point of view.

4. Apparatus according to claim 2 or 3, wherein the control unit (6) is configured to detect on said at least one image (7) at least one color difference (8, 9), the color difference being given by the presence of a lighter color (8) in or within or near a zone (Z1, Z0) of the radioprotective personal protective equipment (21, 21', 21") having another color or predominantly another color (9) and/or in an adjacent zone of the radioprotective personal protective equipment (21, 21', 21"), said other color (9) being darker than the lighter color (8),
for example the difference in colors (8, 9) between adjacent zones (Z0, Z1) of the radioprotective personal protective equipment (21, 21', 21") being an indicator to be taken into account when assessing the presence of significant cracks and/or damages to the radioprotective personal protective equipment (21, 21', 21").

5. Apparatus according to any one of the preceding claims, further comprising an image acquisition and/or recording member (7), configured to acquire, under conditions of use, one or more images (7) of the radioprotective personal protective equipment (21, 21', 21"),
preferably the X-ray detection means (5) including the image acquisition and/or recording member (7).

6. Apparatus according to any one of the preceding claims, comprising a display support (10), for example a screen, on which an operator can view, in particular monitor, whether the radioprotective personal protective equipment (21, 21', 21") is defective or potentially defective based on information provided by the artificial intelligence.

7. Apparatus according to any one of the preceding claims, wherein the control unit (6) implements artificial intelligence operating on a dataset developed by tests previously conducted and in particular validated, preferably the control unit (6) being trained on said dataset.

8. Use of an apparatus (1, 1'), according to any one of the preceding claims, for checking the integrity of one or more radioprotective personal protective equipment (21, 21', 21"),
preferably one or more radioprotective personal protective equipment (21) being designed to protect one or more parts of a user's body, excluding the eyes, and/or one or more radioprotective personal protective equipment (21', 21") being designed to protect a user's eyes.

9. Method for checking, by means of artificial intelligence, the integrity of radioprotective personal protective equipment (21, 21', 21"), the method comprising the following steps:
- emitting X-rays so as to interact with a radioprotective personal protective equipment (21, 21', 21") positioned inside a checking chamber (2),
- detecting the integrity status of the radioprotective personal protective equipment (21, 21', 21") ,
in which the step of detecting the integrity status of the radioprotective personal protective equipment (21, 21', 21") comprises the following steps:
∘ obtaining, by means of artificial intelligence, an index of suitability (IN) for the radioprotective personal protective equipment (21, 21', 21"),
∘ on the basis of the suitability index (IN) obtained, assessing whether the radioprotective personal protective equipment (21, 21', 21") is defective or potentially defective.

10. Method according to claim 9, wherein the step of assessing, on the basis of the suitability index (IN) obtained, whether the radioprotective personal protective equipment (21, 21', 21") is defective or potentially defective, is performed by means of artificial intelligence.

11. Method according to claim 9 or 10, comprising the steps of:
- acquiring and/or recording at least one image (7) or a series of images of the radioprotective personal protective equipment (21, 21', 21") during X-ray emission towards the radioprotective personal protective equipment (21, 21', 21"),
- analysing from a chromatic point of view said at least one image (7) or a series of images of the radioprotective personal protective equipment (21, 21', 21"), the one or more images being acquired during X-ray emission towards the radioprotective personal protective equipment (21, 21', 21"), each image preferably being a fluoroscopic image (7),
- detecting on said at least one image (7) at least one color difference (8, 9) of the radioprotective personal protective equipment (21, 21', 21").

12. Method according to claim 11, wherein:
- the step of acquiring and/or recording at least one image (7) or a series of images provides for acquiring and/or recording at least one fluoroscopic image (7) or a series of fluoroscopic images of the radioprotective personal protective equipment (21, 21', 21") during X-ray emission towards the radioprotective personal protective equipment (21, 21', 21"),
- the step of analysing from a chromatic point of view said at least one image (7) or a series of images of the radioprotective personal protective equipment (21, 21', 21") provides for analysing from a chromatic point of view said at least one fluoroscopic image (7) or a series of fluoroscopic images of the radioprotective personal protective equipment (21, 21', 21").

13. Method according to claim 11 or 12, wherein the color difference (8, 9) detected on said at least one image (7) is due to the presence of a different color (8), e.g. a lighter color, in or within or near an area (Z1, Z0) of the image of the radioprotective personal protective equipment (21, 21', 21") having another color or predominantly another color (9), e.g. a darker color.

14. Method according to claim 11 or 12 or 13, comprising:
o analysing from a chromatic point of view a plurality of images (7) of one or more radioprotective personal protective equipment (21, 21', 21"),
o classifying said plurality of images (7) after analysing them from a chromatic point of view.

15. Method according to claim 11 or 12 or 13 or 14, comprising detecting on said at least one image (7) at least one color difference (8, 9), said color difference (8, 9) being given by the presence of a lighter color (8) either within or close to an area (Z1, Z0) having another color or predominantly another color (9) and/or in an adjacent area of the radioprotective personal protective equipment (21, 21', 21"), said other color (9) being darker than the lighter color (8).
